# EUROPEAN PATENT APPLICATION

(11) **EP 4 292 565 A1**
(43) Date of publication of application: **20.12.2023**
(21) Application number: 22198572.4
(22) Date of filing: 29.09.2022
(51) Int. Cl.: A61C 19/06, A61K 8/18, A61Q 11/00

(54) **SYSTEM FOR ULTRASOUND ENHANCED TOOTH HYPERSENSITIVITY TREATMENT WITH CATIONIC COMPOSITION**

(30) Priority: 17.06.2022 US 202263353045 P
(71) Applicant: Koninklijke Philips N.V., 5656 AG Eindhoven (NL)
(72) Inventor: MAO, Jingliang, Eindhoven (NL)
(74) Representative: Philips Intellectual Property & Standards

(57) **Abstract**

Oral health care systems (10, 100) and methods (200) for the treatment of tooth hypersensitivity, including an ultrasound transducer (14, 114), a dental treatment tray (16, 116) coupled to the ultrasound transducer, and a cationic occlusion composition (18, 118) disposed in the dental tray (16, 116) and including an occlusion agent for treating tooth hypersensitivity. The cationic occlusion composition (18, 118) contacts exposed dentin tubules of the one or more teeth when the dental treatment tray (16, 116) is in use. Ultrasound waves are transmitted into the cationic occlusion composition to enhance diffusion and penetration of the occlusion agent into the exposed dentin tubules of the one or more teeth.

## Description

### FIELD OF THE INVENTION

The present disclosure relates generally to oral health care systems and methods for the treatment of tooth hypersensitivity using ultrasound in combination with cationic occlusion compositions.

### BACKGROUND OF THE INVENTION

Tooth and gum sensitivity are areas of particular concern in oral health care, usually the most prominent concern after tooth cavities. Dentin hypersensitivity is characterized as a sharp short pain arising from exposed dentin, typically in response to an external stimulus such as clinical, thermal, tactile, evaporative or osmotic stimuli. It is often experienced when dentin is exposed to the oral environment because of gingival recession or a loss of enamel.

The most widely accepted explanation for dentin hypersensitivity is provided by the hydrodynamic theory, which suggests that nerve stimulation due to movement of dentinal fluid is the cause of the hypersensitivity. When dentin tubules are exposed to the oral environment, fluid movement through dentin tubules can be sensed by internal tooth nerves. The activation of tooth nerve fibers can cause the feeling of sharp pain. The relationship between dentin hypersensitivity and the patency of dentin tubules has been established in vivo and it has been found that occlusion of the tubules decreases that sensitivity.

In general, the technology and materials developed to treat dentin hypersensitivity can be classified into two treatment categories: Treatment I and Treatment II described below.

Treatment I works by desensitizing the tooth nerves via potassium salts like potassium nitrate, potassium citrate, potassium chloride, which depolarize the excited nerve fibers, thus numbing the pain. This treatment typically provides short-term relief of dentin hypersensitivity.

Treatment II is based on occlusion of the dentin tubules by plugging dentinal tubules or coating the sensitive dentin area with a thin protective layer of material. Occlusion of open tubules blocks the hydrodynamic mechanism. Treatment II may provide a relatively long-term solution.

Most current dentin hypersensitivity treatment products and methods work by blocking the dentinal tubules and preventing dentinal fluid flow. Examples of such materials and methods include the application of specific dentifrices, dentin adhesives, antibacterial agents, aldehydes, resin suspensions, fluoride rinses, fluoride varnishes, amorphous calcium phosphate (ACP), strontium fluorides, oxalates, Nd-YAG laser application, bioactive glass, casein phosphopeptide, and Portland cement.

In the tooth whitening field, U.S. Pat. Publ. No. 2005/0244792 A1 describes a whitening process wherein ultrasound enhances the whitening action of a whitening agent. U.S. Pat. No. 4,116,239 describes a handheld ultrasound instrument that produces a jet of oxygen which may be directed to the site being treated.

In general, the existing products and methods to treat dentin hypersensitivity have one or more of the problems or disadvantages described below.

Existing products typically provide insufficient blockage of open tubules in a depth-wise direction of the tubules as well as in terms of area coverage of the openings of the dentin surface. Existing products generally provide slow diffusion and/or dispersion of the active occlusion ingredients into the open dentin tubules. For example, in products that use an arginine bicarbonate calcium carbonate complex to plug dentinal tubules, arginine binds onto tooth surfaces and recruits calcium and phosphate ions from saliva. Since calcium carbonate has a low solubility in water to produce calcium ions and the tooth dentin microtubules are around 5 µm in diameter, it takes time to build up effective plugging with compounds formed by calcium and phosphate ions inside the dentin tubules. Hence, the efficacy of the existing products is less than desired.

Existing products offer weak or low affinity to dentin and provide unstable remineralization and lower efficiency of occlusion of dentin tubules. For example, calcium and phosphate ions may be released from a pre-formed amorphous calcium phosphate (ACP) gel in the presence of saliva to accelerate the uptake of these bioavailable ions, but the formed compounds lack stability and have low affinity to dentin inside dentin tubules.

Existing products provide hypersensitivity relief only for a limited time. For example, it has been reported that a composition of arginine, bicarbonate and calcium carbonate is burnished onto sensitive teeth following scaling and root planing procedures in a dental clinic, and the reported sensitivity relief lasts for at least 28 days by a single treatment (D. Cummins, Dentin hypersensitivity: from diagnosis to a breakthrough therapy for everyday sensitivity relief, J Clin Dent. 2009;20(1):1-9).

Existing products and methods are inconvenient, costly and/or uncomfortable treatments. Many existing dentin hypersensitivity treatments require dental clinic visits.

In view of the foregoing, there is a persisting need to improve techniques to treat dentin hypersensitivity.

### SUMMARY OF THE INVENTION

Addressing such a need, systems and methods are provided using ultrasound waves to drive various forms of occlusion agents into teeth substances.

The inventive subject matter disclosed herein improves techniques to treat tooth hypersensitivity both from an efficacy and speed perspective. The inventive subject matter enhances diffusion and penetration of occlusion agents, improves the occlusion of dentin tubules by using ultrasound in the tooth hypersensitivity treatment process, and thereby achieves better treatment efficacy, and provides longer dentin hypersensitivity in a convenient and cost-effective way.

The inventive subject matter is directed to an oral health care system for the treatment of tooth hypersensitivity, including an ultrasound transducer configured to emit ultrasound waves at a frequency ranging from about 20 kHz to about 10 MHz, a dental treatment tray including a channel adapted to accommodate one or more teeth of a user. The dental treatment tray is coupled to the ultrasound transducer. A cationic occlusion composition, including an occlusion agent for treating tooth hypersensitivity, is disposed in the channel of the dental treatment tray such that the cationic occlusion composition contacts exposed dentin tubules of the one or more teeth when the dental treatment tray is in use. Ultrasound waves, produced by the ultrasound transducer, are transmitted into the cationic occlusion composition to enhance diffusion and penetration of the occlusion agent into the exposed dentin tubules of the one or more teeth.

In some embodiments, the cationic occlusion composition includes a source of calcium ions; a source of phosphate ions; cationic charged amino acids, proteins, and/or peptides; and deionized water; and wherein the cationic occlusion composition has a pH ranging from about 5.5 to about 9.5. In some embodiments, the cationic occlusion composition is a solution or an emulsion. In other embodiments, the cationic occlusion composition is an on-site instantly formed gel or a pre-formed composition. In some embodiments, the ultrasound transducer can be located externally of an oral cavity of the user when in use. The ultrasound transducer is coupled to the dental treatment tray via a coupling element that transmits ultrasound waves from the ultrasound transducer to the cationic occlusion composition. In further embodiments, the ultrasound transducer includes one or more transducer elements embedded in a side wall of the channel of the dental treatment tray to emit ultrasound waves into the cationic occlusion composition. In some embodiments, the system may include a front reflector and/or a rear reflector configured to direct the ultrasound waves into the cationic occlusion composition. In other embodiments, the system includes a bite plane for interaction with teeth and forming shield to protect the tongue from contact with the cationic occlusion composition. In some embodiments, the ultrasound transducer produces a frequency ranging from about 20 kHz to about 100 kHz and wherein the frequency is centered around about 40kHz to about 50 kHz.

The inventive subject matter is further directed to a method for treating tooth hypersensitivity. The method includes the following steps: providing an ultrasound transducer configured to emit ultrasound waves at a frequency ranging from about 20 kHz to about 10 MHz; providing a dental treatment tray including a channel adapted to accommodate one or more teeth of a user; providing a cationic occlusion composition including an occlusion agent for treating tooth hypersensitivity; disposing the cationic occlusion composition in the channel of the dental treatment tray and positioning the dental treatment tray over the one or more teeth of the user such that the cationic occlusion composition contacts exposed dentin tubules of the one or more teeth when the dental treatment tray is in use; coupling the ultrasound transducer to the dental treatment tray; and activating the ultrasound transducer to produce ultrasound waves and transmitting the ultrasound waves into the cationic occlusion composition to enhance diffusion and penetration of the occlusion agent into the exposed dentin tubules of the one or more teeth. The method can apply any of the system embodiments described herein.

These and other aspects of the disclosure will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the inventive subject matter.
FIG. 1 shows examples of types of ultrasound transducers that can be modified as internal or external ultrasound transducer components for use in the inventive subject matter.
FIG. 2 is a schematic top view of an ultrasound assisted tooth hypersensitivity treatment system with cationic composition and an external ultrasound transducer.
FIG. 3 is a schematic top view of an ultrasound assisted tooth hypersensitivity treatment system with cationic composition and an internal ultrasound transducer.
FIG. 4 is a high-level flow chart of an exemplary method for treating tooth hypersensitivity.

### DETAILED DESCRIPTION OF EMBODIMENTS

Oral health care systems and methods for the treatment of tooth hypersensitivity are disclosed. The inventive subject matter combines the application of a cationic composition and the application of therapeutic ultrasound waves thereon to achieve a synergistic effect of enhanced diffusion and penetration of occlusion agents into dentin tubules. The inventive subject matter not only reduces the hypersensitivity treatment time as ultrasound assisted diffusion and penetration of occlusion agents helps the occlusion process and optimizes treatment efficacy, it also achieves a longer dentin hypersensitivity relief effect in a convenient way and ensures a more cost-effective hypersensitivity treatment for sensitive teeth.

It is known that dentin or tooth surface is negatively charged in nature. Chemically, dentin is composed, by weight, of approximately 20% of organic material, approximately 75% of inorganic material, and approximately 5% water. The organic material is primarily collagen, which functions as a binder in the dentin structure. The inorganic material includes calcium phosphate.

In healthy teeth, saliva is naturally effective in reducing dentin hypersensitivity by supplying and carrying calcium and phosphate ions into the open dentin tubules to gradually occlude them and to form a surface protective layer consisting of precipitate of salivary glycoproteins with calcium phosphate. For people having dentin hypersensitivity, the affected tooth dentin exposes numerous microtubules of around 5 µm in diameter. The microtubules allow small molecular size fluid or particles to migrate inside the dentin structure. It is beneficial to have a composition of a dentin occluding material close or similar to dentin's natural composition, i.e., being made of calcium phosphates; amino acids, peptides, or proteins; and water.

An approach that speeds up the diffusion and penetration of occlusion agents into tooth dentin via ultrasound assistance is beneficial for the treatment. A cationic charged, instantly formed ACP gel is desired in terms of freshness, binding affinity, and occlusion efficiency of dentin tubules. The synergistic effect is achieved by using ultrasound-enhanced diffusion, the interaction principle of charged particles in physics that opposites charges attract each other and like charges repel each other, and by using cationic occlusion agents.

In physical chemistry, diffusion is the net movement of molecules from a region of higher concentration to a region of lower concentration. Diffusion is driven by a gradient in chemical potential of the diffusing species. In tooth occlusion, the gradient is a concentration gradient that reflects change in the value of occlusion species concentration with the change in distance.

In physics, ultrasound is a mechanical wave propagating in a longitudinal direction. It is transmitted in a straight line and it can be focused. Ultrasound waves obey laws of reflection and refraction. In a real-world application, ultrasound cannot be transmitted through a gaseous medium, like air or lung tissue. The stiffer the tissue, the faster will the ultrasound travel in that medium. Ultrasound can help the penetration of small particles into biofilms, enhance transdermal delivery of drugs, and enhance diffusion of whitening agents into the dentin via enamel.

In applications where ultrasound is not used, the occlusion species slowly reach the dentin by diffusion and the depth of diffusion inside the dentin is not ideal enough. This diffusion depends on the random "walk" of the occlusion species, and results in mixing or mass transport of the occlusion species without requiring directed bulk motion.

As used herein, the term "enhanced diffusion" refers to having an increase in the release of the occlusion agent leading to an increase in the uptake of the occlusion agent into the exposed dentin tubules of the teeth. Ultrasound facilitates the diffusion process by increasing the Brownian-motion like movement of molecules, thus, the opportunity of negative teeth dentin surface meeting cationic occlusion agent molecules is significantly increased. During ultrasound-aided occlusion, ultrasound speeds up the diffusion and penetration of occlusion species into the dentin by acoustic oscillations, intermittent retention due to interaction with the pore walls of the enamel and dentin, and some cavitation. Safe ultrasound cavitation can disrupt the structure of occlusion gels and releases the occlusion species fast while agitating the enamel matrix and make it more permeable for the occlusion species.

The application of ultrasound leads to significant diffusion increase or enhancement. For example, in tooth whitening procedures, empirical results show ultrasound can make it take as short as 4 minutes for a peroxide species to reach the dentin via ultrasound-aided diffusion, compared to the more than 10 minutes diffusion time required in non-ultrasound-assisted tooth whitening approaches. The diffusion rate of occlusion agents into dentin determines the occlusion rate and treatment results. Hence, synergistic conditions using ultrasound achieve high plugging efficiency of exposed dentinal tubules in a short amount of time which is significantly shorter than non-synergistic conditions where ultrasound is not used. Compared to previous known dentin occlusion methods and products, the inventive subject matter uses the synergistic potential of ultrasound and cationic occlusion agents in one system to accelerate diffusion of occlusion ions and molecules, thereby enhancing the tooth dentin occlusion process.

The oral health care system uses a cationic occlusion composition including an occlusion agent. The term "occlusion agent" refers to one or more compounds that provide the desired effect of hypersensitivity relief. Examples of a suitable occlusion agents include calcium ions; phosphate ions; amino acids, peptides, or proteins; and water with cationic charged amino acids, peptides, and/or proteins at pH of about 5.5 to about 9.5. A cationic occlusion composition can be a pre-formed composition or an instantly on-site formed composition. It could be a gel or a solution, depending on application requirement. Specific examples of occlusion agents are provided in the cationic composition examples set forth below.

As used herein, the terms "instant" and "instantly" refer to the short time from the time of mixing the components to the time of formation of the finished gel, which is generally less than about 5 seconds.

As used herein, the term "gel" refers to a homogenous solid aqueous gel-like material, such as a hydrogel having water-swellable polymeric matrices that can absorb a substantial amount of water.

An oral health care system for the treatment of tooth hypersensitivity according to the disclosure includes an ultrasound transducer, a dental treatment tray, and a cationic occlusion composition. The cationic occlusion composition is disposed in a channel of the dental treatment tray such that the cationic occlusion composition contacts exposed dentin tubules of the tooth when the dental treatment tray is in use. Ultrasound waves, produced by the ultrasound transducer, are transmitted into the cationic occlusion composition to enhance diffusion and penetration of the occlusion agent into the exposed dentin tubules of the one or more teeth.

An ultrasound power source, or ultrasonic generator, with an adjustable frequency range of 20 kHz to 3.0 MHz or higher can be used. The electrical output from the power source is converted into mechanical vibration through a transducer. The intensity of the produced ultrasound waves should be suitable for teeth hypersensitivity treatment applications. For safe human use, a power source producing a frequency of less than 10 MHz is preferred, particularly power sources producing a frequency range of 20 kHz to 100 kHz and centered around 40 kHz to 50 kHz are of interest.

A dental treatment tray can hold the cationic occlusion composition that surrounds the one or more teeth or dental area to be treated. In some embodiments, a dental treatment tray can be adapted to hold teeth of an upper dental arch, a lower dental arch, or a portion of the upper or lower dental arches.

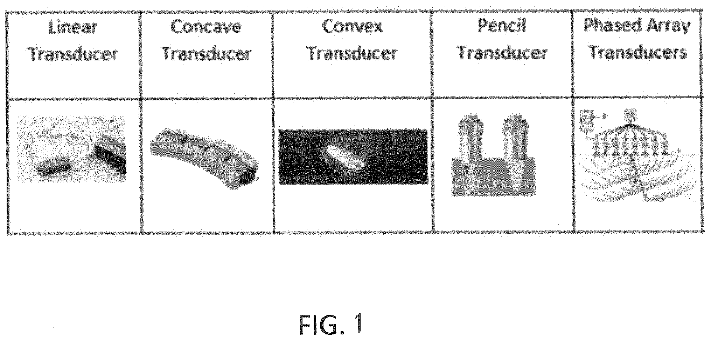

FIG. 1 illustrates examples of types of ultrasound transducers that can be modified as internal or external ultrasound transducer components according to the disclosure, such as a linear transducer, a concave or convex transducer, a pencil transducer, and/or phased array transducers. In further embodiments, ultrasound transducers or transducer arrays can be embedded in the dental treatment tray.

Depending on how to the individual parts of the system are arranged, the final configuration of the system varies accordingly. Details of the oral health care system and its components are provided in the following detailed descriptions. The actual size/shape/appearance/necessity of each component can be minimized, added, or modified to make it a fit and attractive device for commercial purpose.

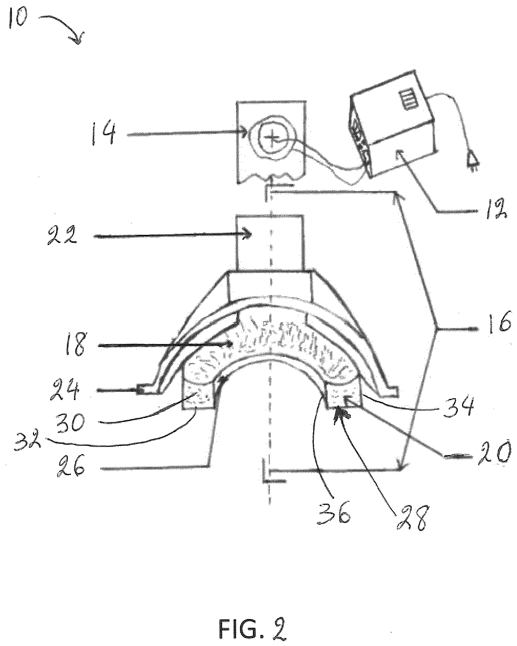

FIG. 2 is schematic top view of an embodiment of a system 10 wherein an ultrasound power source 12 is coupled to an external transducer 14. In other embodiments, multiple external transducer elements can used to produce the ultrasound waves. A dental treatment tray 16 is configured to hold a cationic composition 18 such that it can be applied to a user's teeth or other area desired to be treated. Dental treatment tray 16 includes a body 30 having a base 32 and side walls 34, 36 that define a channel 20, sized and shaped to correspond to the curvature of a dental arch, or at least a portion thereof. Channel 20 surrounds the one or more teeth of the user when dental treatment tray 16 is in use such that cationic composition 18, which is disposed in channel 20, contacts the desired treatment area of teeth and/or gum. Channel 20 is adapted to hold cationic composition 18, in solution form or in gel form. Dental treatment tray 16 can be made of a generally fluid impervious material.

Coupling element 22 connects external transducer 14 to dental treatment tray 16 to transfer ultrasound waves from external transducer 14 to dental treatment tray 16 and further onto cationic composition 18. Coupling element 22 includes ultrasound connectors and cables serving to conduct externally generated ultrasound waves into the interior of tray channel 20 and into cationic occlusion composition 18. Coupling element 22 can further include a rod and/or a plate that projects exteriorly from dental treatment tray 16 functioning as a handle to assist positioning of dental treatment tray 16 in the mouth of a user.

To allow effective distribution of ultrasound waves onto cationic composition 18, system 10 is equipped with a front reflector 24 and a rear reflector 26. Front reflector 24 has a parabolic shape configured to distribute and transmit the ultrasound waves into cationic composition 18. Rear reflector 26 also has a parabolic shape to reflect ultrasound waves back into cationic composition 18. A bite plane 28 is formed by base 32 of dental treatment tray 16 providing a surface for teeth to rest on and forming a tongue shield to protect the tongue from cationic composition 18.

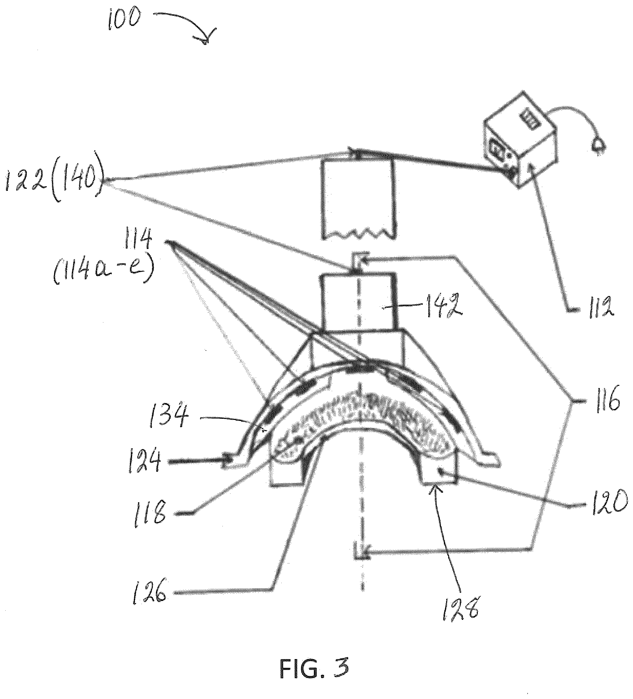

In other embodiments, an oral health care system can include an intra-oral ultrasound transducer array to transmit the ultrasound waves into the cationic occlusion composition. FIG. 3 illustrates an embodiment of a system 100 wherein an ultrasound power source 112 and internal ultrasound transducer 114 are used. Transducer 114, in the form of five transducer elements 114a, 114b, 114c, 114d, 114e, is embedded in dental treatment tray 116. Transducer elements 114a-e are evenly dispersed inside a vertical side wall 134 of channel 120 to cover the area to be treated and are configured to produce ultrasound waves that are transmitted onto cationic composition 118. In other embodiments, the number of transducer elements can vary depending on the application. In further embodiments, transducer elements can have different shapes with different widths, heights, or radii.

System 100 further includes of a front reflector 124 and a rear reflector 126. A bite plane 128 connects with front reflector 124 and rear reflector 126. Front reflector 124, rear reflector 126 and bite plane 128 have similar functionalities as described for the embodiment above. A connector element 122 couples power source 112 to internal ultrasound transducer 114. Connector element 122 includes wiring 140 to couple power supply 112 to internal ultrasound transducer 114, and further includes a positioner 142 serving as a handle that allows proper positioning of dental treatment tray 116 into the mouth.

Cationic compositions for tooth hypersensitivity treatment can be formulated to provide a synergistic effect with the application of ultrasound waves. A cationic composition for tooth hypersensitivity treatment can be formulated by utilizing well-designed formulation techniques and safe/non-toxic ingredients. Table 1 and Table 2 below show examples of different cationic compositions for tooth hypersensitivity treatment formulas. Table 1 shows an exemplary formulation for a composition in solution form. Table 2 shows an exemplary formulation for a composition in gel form.

**TABLE 1**

| Part 1 | |
|---|---|
| Ingredient | % w/w in Part 1 |
| DI Water | 94.235 |
| Calcium Nitrate | 0.750 |
| Biotin, USP | 0.012 |
| Sodium Saccharin USP/FCC | 0.001 |
| Potassium Nitrate | 5.000 |
| Phosphoric Acid, 50% | Q.S to pH 6.0- 6.5 |

| Part 2 | |
|---|---|
| Ingredient | % w/w in Part 2 |
| DI Water | 80.416 |
| Avidin, USP | 0.500 |
| Arginine | 9.000 |
| Disodium Phosphate | 0.750 |
| Potassium Nitrate | 5.000 |
| Glycerine | 4.000 |
| Sodium Saccharin USP/FCC | 0.001 |
| Sodium Fluoride USP | 0.290 |
| Phosphoric Acid, 50% | Q.S to pH 9.0- 9.5 |

For quality control purposes, the pH of the mixture of Part 1 and Part 2 at 1:1 (v/v) mixing ratio should be within range of pH 6.5-8.0, preferably approximately pH 7.0-7.5.

**TABLE 2**

| **Part A** | |
|---|---|
| **Ingredient** | % **w/w in Part A** |
| DI Water | 88.826 |
| Calcium Nitrate | 0.750 |
| Phosphoric Acid, 75% | 0.002 |
| ACULYN^{™} 28 | 10.000 |
| Sodium Lauryl Sulfate (STEPANOL^{®} WA-100 NF/USP) | 0.222 |
| Natural Peppermint Oil | 0.200 |

| **Part B** | |
|---|---|
| **Ingredient** | % w/w **in Part b** |
| DI Water | 76.479 |
| Sodium Hydroxide | 0.328 |
| Arginine | 8.000 |
| Disodium Phosphate | 0.750 |
| Potassium Nitrate | 10.000 |
| Glycerine | 4.000 |
| Sodium Saccharin USP/FCC | 0.001 |
| Sodium Fluoride USP | 0.442 |

The mix ratio of Part A and Part B in Table 3 is 1:1 (v/v) to create a cationic arginine ACP gel that is formed instantly. The gel can be made on-site, i.e., at the location of use shortly prior to administration, to preserve freshness and effectiveness. The formulation can be accomplished by a pH change of the mixture when a pH sensitive thickener, such as ACULYN^{™} 28, is used. A pH manipulation is conducted by raising the pH of the final mixture to an alkaline pH, in particular a pH of about 7.0 to about 12.0, when a predetermined amount of one acidic component with pH sensitive thickener is mixed with an amount of another alkaline component via a mixing tip or in a mixing chamber. The pH sensitive thickener is capable of increasing the viscosity of the composition by subjecting it to an increasing pH, in particular a pH in the range of 7.0 to 12.0. The thickener is compatible with one or more of the basic amino acids/peptides, phosphate ions, calcium ions, and/or acidic amino acids.

In some embodiments, the system can be implemented as a treatment gel product that is applied at a dental office. In other embodiments, the system can be implemented as "take-home" or retail type product for dentin hypersensitivity treatment, for example as a kit including a cationic composition and an ultrasound device. In further embodiments, the composition may be used to treat tooth cavities due to the same mechanism and strong affinity to tooth enamel.

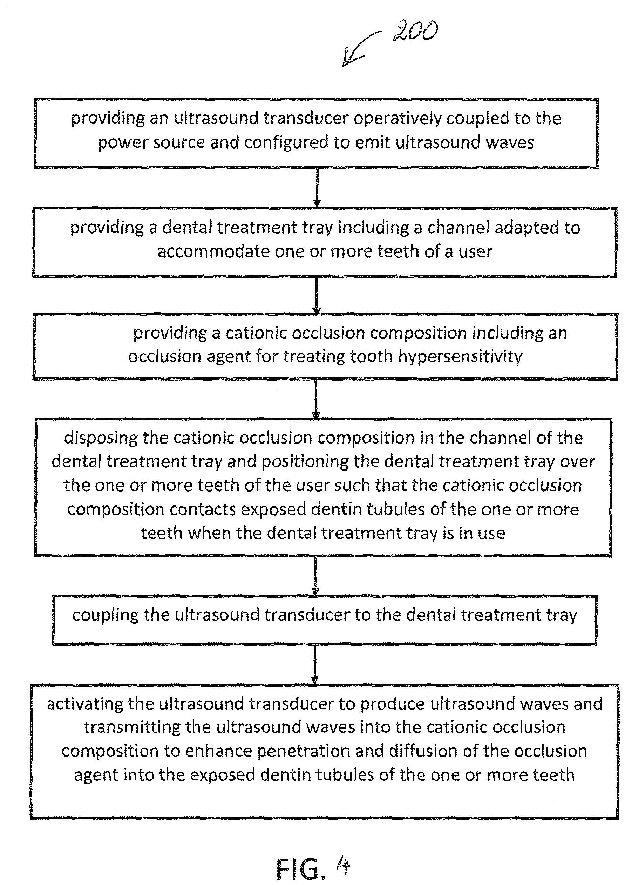

The inventive subject matter further contemplates a method for treating tooth hypersensitivity. FIG. 4 illustrates the steps of a method 200, including providing an ultrasound transducer configured to emit ultrasound waves at a frequency ranging from about 20 kHz to about 10 MHz, providing a dental treatment tray including a channel adapted to accommodate one or more teeth of a user, providing a cationic occlusion composition including an occlusion agent for treating tooth hypersensitivity, disposing the cationic occlusion composition in the channel of the dental treatment tray and positioning the dental treatment tray over the one or more teeth of the user such that the cationic occlusion composition contacts exposed dentin tubules of the one or more teeth when the dental treatment tray is in use, coupling the ultrasound transducer to the dental treatment tray, and activating the ultrasound transducer to produce ultrasound waves and transmitting the ultrasound waves into the cationic occlusion composition to enhance diffusion and penetration of the occlusion agent into the exposed dentin tubules of the one or more teeth. Method 200 can use the systems, arrangements, and compositions of any of the embodiments described above.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one." In addition, singular references do not exclude a plurality. Thus, references to "a", "an", "first", "second" etc. do not preclude a plurality.

Reference signs in the claims are provided merely as a clarifying example shall not be construed as limiting the scope of the claims in any way.

The phrase "and/or," as used herein in the specification and in the claims, should be understood to mean "either or both" of the elements so conjoined, *i.e.,* elements that are conjunctively present in some cases and disjunctively present in other cases. Multiple elements listed with "and/or" should be construed in the same fashion, *i. e.,* "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, *i*.*e.*, the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (*i.e.* "one or the other but not both") when preceded by terms of exclusivity, such as "either," "one of," "only one of," or "exactly one of."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, *i.e*., to mean including but not limited to.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. An oral health care system (10, 100) for the treatment of tooth hypersensitivity, comprising:
an ultrasound transducer (14, 114) configured to emit ultrasound waves at a frequency ranging from about 20 kHz to about 10 MHz;
a dental treatment tray (16, 116) including a channel (20, 120) adapted to accommodate one or more teeth of a user, the dental treatment tray is coupled to the ultrasound transducer;
a cationic occlusion composition (18, 118) including an occlusion agent for treating tooth hypersensitivity, the cationic occlusion composition (18, 118) is disposed in the channel (20, 120) of the dental treatment tray (16, 116) such that the cationic occlusion composition contacts exposed dentin tubules of the one or more teeth when the dental treatment tray is in use; and
wherein ultrasound waves, produced by the ultrasound transducer (14, 114), are transmitted into the cationic occlusion composition (18, 118) to enhance diffusion and penetration of the occlusion agent into the exposed dentin tubules of the one or more teeth.

2. The oral health care system of claim 1, wherein the cationic occlusion composition includes a source of calcium ions; a source of phosphate ions; cationic charged amino acids, proteins, and/or peptides; and deionized water; and wherein the cationic occlusion composition has a pH ranging from about 5.5 to about 9.5.

3. The oral health care system of claim 1, wherein the cationic occlusion composition is a solution or an emulsion.

4. The oral health care system of claim 1, wherein the cationic occlusion composition is an on-site instantly formed gel.

5. The oral health care system of claim 1, wherein the cationic occlusion composition is a pre-formed composition.

6. The oral health care system of claim 1, wherein the ultrasound transducer is located externally of an oral cavity of the user when in use, and wherein the ultrasound transducer is coupled to the dental treatment tray via a coupling element (22) that transmits ultrasound waves from the ultrasound transducer to the cationic occlusion composition.

7. The oral health care system of claim 1, wherein the ultrasound transducer (114) includes one or more transducer elements (114a, 114b, 114c, 114d, 114e) embedded in a side wall (134) of the channel (120) of the dental treatment tray to emit ultrasound waves into the cationic occlusion composition.

8. The oral health care system of claim 1, further comprising a front reflector (24, 124) configured to direct the ultrasound waves into the cationic occlusion composition.

9. The oral health care system of claim 1, further comprising a rear reflector (26, 126) configured to reflect the ultrasound waves into the cationic occlusion composition.

10. The oral health care system of claim 1, further comprising a bite plane (28, 128) for interaction with teeth and forming shield to protect the tongue from contact with the cationic occlusion composition.

11. The oral health care system of claim 1, wherein the ultrasound transducer (14, 114) produces a frequency ranging from about 20 kHz to about 100 kHz and wherein the frequency is centered around about 40kHz to about 50 kHz.

12. A method (200) for treating tooth hypersensitivity, the method comprising:
providing an ultrasound transducer (14, 114) configured to emit ultrasound waves at a frequency ranging from about 20 kHz to about 10 MHz;
providing a dental treatment tray (16, 116) including a channel (20, 120) adapted to accommodate one or more teeth of a user;
providing a cationic occlusion composition (18, 118) including an occlusion agent for treating tooth hypersensitivity;
disposing the cationic occlusion composition (18, 118) in the channel (20, 120) of the dental treatment tray (16, 116) and positioning the dental treatment tray over the one or more teeth of the user such that the cationic occlusion composition contacts exposed dentin tubules of the one or more teeth when the dental treatment tray is in use;
coupling the ultrasound transducer (14, 114) to the dental treatment tray (16, 116); and
activating the ultrasound transducer (14, 114) to produce ultrasound waves and transmitting the ultrasound waves into the cationic occlusion composition (18, 118) to enhance diffusion and penetration of the occlusion agent into the exposed dentin tubules of the one or more teeth.

13. The method of claim 12, wherein the cationic occlusion composition includes a source of calcium ions; a source of phosphate ions; cationic charged amino acids, proteins, and/or peptides; and deionized water; and wherein the cationic occlusion composition has a pH ranging from about 5.5 to about 9.5.

14. The method of claim 12, wherein the ultrasound transducer is located externally of an oral cavity of the user when in use, and wherein the ultrasound transducer is coupled to the dental treatment tray via a coupling element (22) that transmits ultrasound waves from the ultrasound transducer to the cationic occlusion composition.

15. The method of claim 12, wherein the ultrasound transducer includes one or more transducer elements embedded in a wall of the channel of the dental treatment tray to emit ultrasound waves into the cationic occlusion composition.
